Europäisches Patentamt

European Patent Office

Office européen des brevets

(19)

(11) **EP 1 019 319 B1**

(12) **FASCICULE DE BREVET EUROPEEN**

(45) Date de publication et mention
de la délivrance du brevet:
**27.11.2002   Bulletin 2002/48**

(51) Int Cl.[7]: **C01B 21/09**, C07D 209/08,
C07D 209/52

(21) Numéro de dépôt: **98946522.4**

(22) Date de dépôt: **29.09.1998**

(86) Numéro de dépôt international:
**PCT/FR98/02081**

(87) Numéro de publication internationale:
**WO 99/016707 (08.04.1999 Gazette 1999/14)**

(54) **PROCEDE DE SYNTHESE DE SOLUTION DE CHLORAMINE HAUTE TENEUR**

VERFAHREN ZUR HERSTELLUNG VON LÖSUNGEN MIT EINEM HOHEN CHLORAMIN-GEHALT

SYNTHESIS METHOD FOR SOLUTION WITH HIGH GRADE CHLORAMINE

(84) Etats contractants désignés:
**AT BE CH DE DK ES FI FR GB GR IE IT LI LU NL
PT SE**

(30) Priorité:  **30.09.1997  FR 9712109**

(43) Date de publication de la demande:
**19.07.2000   Bulletin 2000/29**

(73) Titulaire: **LES LABORATOIRES SERVIER
92200 Neuilly sur Seine (FR)**

(72) Inventeurs:
• **DELALU, Henri
F-69002 Lyon (FR)**

• **PEYROT, Laurent
F-69330 Meyzieu (FR)**
• **ELKHATIB, Mazen
F-69100 Villeurbanne (FR)**
• **COUNIOUX, Jean-Jacques
F-69003 Lyon (FR)**
• **COHEN, Armand
F-76210 Bolbec (FR)**

(56) Documents cités:
**FR-A- 2 610 321          FR-A- 2 663 324
US-A- 3 254 952**

## Description

La présente invention concerne un procédé de préparation de chloramine à haute teneur par action d'eau de Javel commerciale à 100 degrés chlorométriques sur une solution d'ammoniac en présence de chlorure d'ammonium. Selon ce procédé, la chloramine est obtenue à une teneur supérieure ou égale à 2 mol L$^{-1}$, soit supérieure ou égale à 10,3 %. Il est utilisable en continu ou en discontinu. Outre ses applications comme agent blanchissant, désinfectant et bactéricide, la monochloramine intervient dans de nombreuses réactions comme intermédiaire de synthèse. En particulier, elle constitue le réactif fondamental de la synthèse d'hydrazines d'intérêt pharmaceutique par le procédé Raschig. C'est ainsi que la N-amino-2-méthylindoline et le N-amino-3-azabicyclo[3.3.0]octane sont préparés en faisant réagir NH$_2$Cl sur la 2-méthylindoline et le 3-azabicyclo[3.3.0]octane selon les procédés décrits dans les brevets EP 462 016 et EP 277 267.

Cette méthode de préparation suscite aujourd'hui un grand intérêt, compte tenu de son caractère peu polluant par rapport aux préparations antérieures faisant appel aux dérivés N-nitrosés. Cependant, elle présente des inconvénients liés à la faible teneur en hydrazine dans les liqueurs réactionnelles qui ne dépassent pas en général 2 à 4 %. Cette limitation est liée principalement à la grande dilution des réactifs et en particulier de l'hypochlorite de sodium, ce qui entraîne des opérations d'extraction délicates et onéreuses. Il en résulte que, dans le meilleur des cas, la concentration instantanée de la chloramine dans le réacteur n'excède par 1 mol L$^{-1}$ (5,15 %).

Compte tenu de la grande instabilité de NH$_2$Cl en solution aqueuse, aucune tentative de synthèse en milieu plus concentré n'a été effectuée. Cette instabilité est liée à une compétition entre des réactions d'hydrolyse et de dismutation autocatalytique qui conduisent à une acidification rapide du milieu et à la formation majoritaire de dichloramine et de chlorure d'azote. La dégradation, en milieu non tamponné, peut entraîner rapidement l'ébullition de la solution. L'obtention d'un titre plus élevé en monochloramine exige la mise en oeuvre d'un nouveau réactif à haut degré chlorométrique et la recherche de conditions opératoires appropriées.

La présente invention permet au moins de doubler le titre en NH$_2$Cl en partant de solutions d'eau de Javel de l'ordre de 100 degrés chlorométriques et en opérant dans des conditions compatibles avec l'obtention de chloramine haute teneur (H.T.). Ce nouveau procédé permet, à partir d'un même volume unitaire d'hypochlorite, de doubler la productivité en hydrazine et de réaliser des économies de matières premières et d'énergie au niveau des opérations de synthèse, de recyclage des réactifs et de distillation.

La réaction d'oxydation de l'ammoniac par l'hypochlorite de sodium étant exothermique, elle doit être effectuée à basse température afin de limiter les réactions de dégradation. Il est donc nécessaire de refroidir préalablement les réactifs avant leur injection. Par exemple, pour une solution d'eau de Javel à 48 degrés chlorométriques (point de cristallisation = - 21,4°C), la température au sein du réacteur ne doit pas être supérieure à -5°C. Cette limite doit être plus basse pour une teneur en NaOCl deux fois plus élevée. Contrairement à l'hypochlorite à 2,14 mol L$^{-1}$ (48° chl.), une solution d'eau de Javel à 100 degrés chlorométriques cristallise en milieu agité à 14°C. La cristallisation demeure néanmoins difficile sans amorçage. Ainsi, en milieu tranquille, on observe un très net retard à la cristallisation et il est alors possible de maintenir en milieu monophasé pendant plusieurs heures des solutions d'eau de Javel H.T. dans des enceintes en verre à -20°C. Cependant, malgré le peu d'aptitude de la solution à cristalliser, il est nécessaire pour une synthèse industrielle en continu d'utiliser l'eau de Javel H.T. vers 14°C afin d'éviter une éventuelle prise en masse dans les canalisations.

Comme dans le procédé de préparation de NH$_2$Cl à partir d'hypochlorite de sodium à 48 degrés chlorométriques, il est indispensable d'introduire dans le réactif ammoniacal une quantité d'un produit accepteur capable de neutraliser totalement les ions hydroxyle formés in-situ par la réaction :

$$ClO^- + NH_3 \rightarrow NH_2Cl + HO^-$$

L'accepteur peut être un acide, un sel acide soluble dans l'eau ou un sel neutre soluble dans l'eau. Toutefois, il est préférable d'utiliser un sel d'ammonium de type $(NH^+_4)_\alpha H^+_\beta A^{(\alpha+\beta)-}$ de manière à tamponner le milieu réactionnel autour du pKa de $NH^+_4/NH_3$, d'éliminer les ions HO$^-$ et de maintenir la concentration en ammoniac libre constante :

$$NH_4^+ + HO^- \rightarrow NH_3 + H_2O$$

D'autre part, le mélange mixte ammoniacal doit être refroidi bien en dessous de -10°C de manière à absorber la chaleur dégagée et à compenser l'échauffement lié à l'injection de l'hypochlorite à une température supérieure à celle de sa cristallisation. Dans ces conditions, les frigories apportées principalement par la solution ammoniacale évitent d'utiliser une eau de Javel à l'état de surfusion et assurent ainsi la synthèse en milieu homogène.

Ces contraintes imposent la recherche de nouvelles combinaisons ammoniacales à bas point de cristallisation compris entre -20°C et -30°C. La composition de ces mélanges est imposée par la teneur des solutions d'hypochlorite. Ces dernières titrent environ 100 degrés chlorométriques. ce qui signifie qu'un litre, dans les conditions normales, libère 100 litres de chlore sous l'action du chlorure d'hydrogène. La concentration molaire en NaOCl est alors de 4,46 mol L$^{-1}$. Contrairement aux ex-

traits à 48 degrés chlorométriques, les titres en NaCl et NaOCl ne sont pas en proportions équimoléculaires.

Pour obtenir des rendements quantitatifs, les conditions de synthèse doivent répondre aux critères suivants :

♦ L'eau de Javel H.T. peut être utilisée à l'état sous-refroidi mais afin de prévenir toute amorce de cristallisation, il est préférable de l'injecter à une température supérieure ou égale à 15°C. Celle-ci est relativement stable et perd environ par 24 heures 1,3 degrés à 17°C ou 0,47 degré à 10°C.

♦ La teneur globale en acide de Lewis doit être suffisante pour neutraliser au moins 90 % des ions hydroxyle résultants de la réaction $NH_3/NaOCl$.

♦ Le pH doit être maintenu entre 10 et 12, de préférence au voisinage de 10,5.

♦ Le rapport [ammoniac total]/[ClO⁻] doit se situer entre 2 et 5 (de préférence 2,7) de manière à stabiliser la chloramine formée.

♦ Le point de cristallisation du mélange ternaire $(NH^+_4)_\alpha H^+_\beta A^{(\alpha+\beta)-}/NH_3/H_2O$ doit être compris entre -20 et -30°C de manière à pallier le déficit de frigories lié à l'injection du réactif chloré à T ≥ 15°C dans le cadre d'une synthèse en milieu monophasique.

♦ La température au sein du réacteur doit se situer dans l'intervalle -5 à -20°C et de préférence autour de -15°C.

Pour élaborer la chloramine, un grand nombre d'acides (HCl, $H_2SO_4$, etc), de sels neutres ($CaCl_2$, $MgCl_2$, $ZnSO_4$, etc) et de sels acides ($NaH_2PO_4$, $NH_4HCO_3$, $NH_4Cl$, $NH_4NO_3$, etc) peuvent être utilisés. A priori, certains sont très intéressants puisqu'ils présentent dans la même molécule plusieurs acidités. En revanche, très peu sont solubles et précipitent en dessous de 0°C. A ce stade, les sels ammoniacaux sont les plus avantageux car ils présentent une plus grande solubilité qui s'accroît avec la teneur en ammoniac dans le milieu.

Dans les conditions de synthèse définies précédemment, l'exploitation des diagrammes polythermiques faisant intervenir l'ion $NH^+_4$ montre que les combinaisons ammoniacales répondant aux critères précédents impliquent l'utilisation de nitrate d'ammonium : $NH_3-NH_4Cl-NH_4NO_3-H_2O$ ou $NH_3-NH_4NO_3-H_2O$. Ces mélanges mixtes permettent de travailler en milieu homogène jusqu'à une température de -30°C. En l'absence de $NH_4NO_3$, la préparation de la chloramine H.T. ne peut être réalisée qu'en batch. En effet, en début de réaction, une fraction du sel de neutralisation est insoluble puis le mélange devient rapidement homogène par dilution et neutralisation des ions HO⁻.

Ainsi selon l'invention, la préparation en continu de la chloramine en milieu homogène doit être effectuée en présence d'une combinaison ammoniacale à base de nitrate d'ammonium $(NH_3/(NH^+_4)_\alpha H^+_\beta A^{(\alpha+\beta)-}/NH_4NO_3/H_2O)$, de préférence $NH_3/NH_4NO_3/H_2O$ ou $NH_3/NH_4Cl/NH_4NO_3/H_2O$, la préparation en discontinu de la chloramine doit être réalisée avec des mélanges de type $NH_3/(NH^+_4)_\alpha H_\beta^+ A^{(\alpha+\beta)-}/H_2O$, de préférence $NH_3/NH_4Cl/H_2O$ qui peuvent être partiellement insolubles au cours de l'addition du réactif chloré.

A titre non limitatif, les exemples suivants illustrent la mise en oeuvre en discontinu et en continu du procédé de l'invention :

## EXEMPLE I :

La réaction est effectuée dans un réacteur cylindrique à double paroi en verre borosilicaté maintenu à -20°C par circulation d'un fluide thermostatique. La solution ammoniacale titre 7,2 mol $L^{-1}$ en $NH_3$ et 4,76 mol $L^{-1}$ en $NH_4Cl$, ce qui correspond à la composition pondérale suivante : 12,1 % $NH_3$, 25,2 % $NH_4Cl$, 62,7 % $H_2O$. 30 mL de ce mélange sont ensuite introduits dans l'enceinte sous agitation jusqu'à ce que l'équilibre thermique soit atteint. La solution d'hypochlorite de sodium (104,4 degrés chlorométriques ; 4,64 mol $L^{-1}$) à l'état sous-refroidi est ensuite versée avec un débit régulier (durée d'écoulement 10 minutes) de manière à ce que la température au sein du réacteur ne dépasse pas -10°C. Le point représentatif du mélange ammoniacal se situant, en début de réaction, à l'intérieur du domaine diphasique liquide + $NH_4Cl$ du diagramme ternaire $NH_3-NH_4Cl-H_2O$ (isotherme-20°C), 29,3 % de la quantité initiale de chlorure d'ammonium sont précipités. Au cours de l'addition de NaOCl, le mélange devient rapidement homogène par dilution et élimination des ions hydroxyle. On obtient au terme de la réaction une solution de chloramine titrant 2,18 mol $L^{-1}$ (11,2 %) ce qui correspond à un rendement de 98 % par rapport à l'hypochlorite. Dans les conditions décrites par cette expérience, la solution de $NH_2Cl$ haute teneur formée est relativement stable à -20°C et ne perd que 1,4 % après 15 minutes.

## EXEMPLE II :

La réalisation de la synthèse dans les mêmes conditions mais sans précipitation initiale de $NH_4Cl$ (milieu homogène) impose une diminution de la teneur en chlorure d'ammonium. Comme le montre la courbe de saturation du ternaire, il est nécessaire d'augmenter le titre en $NH_3$ (injection de $NH_3$ gazeux), de manière à dissoudre $NH_4Cl$. En partant, par exemple, d'une solution commerciale d'ammoniac à 32 %, la quantité maximale dissoute de $NH_4Cl$ permise par l'isotherme -20°C correspond à la composition globale suivante : 25.3 % NH3 (14,25 mol $L^{-1}$), 20,9 % $NH_4Cl$ (3,74 mol $L^{-1}$). On obtient une solution de $NH_2Cl$ concentrée titrant 1,99

mol L$^{-1}$ (10,2 %) soit un rendement de 90 % par rapport à NaOCl. Cette légère diminution du rendement est due au fait que 16 % de la réaction ont été réalisés en milieu non tamponné.

## EXEMPLE III :

Cet essai a été conduit en utilisant une solution d'hypochlorite non plus à l'état sous refroidi mais à une température supérieure à son point de cristallisation (+15°C). 30 ml d'une solution mixte ammoniacale titrant 12,4 mol L$^{-1}$ en NH$_3$ (21,2 %) et 5 mol L$^{-1}$ en NH$_4$Cl (26,8 %) sont introduits dans une enceine maintenue à -25°C. Un volume équivalent d'eau de Javel (103,8 degrés chlorométriques ; 4,63 mol L$^{-1}$) est ensuite versé goutte à goutte (durée d'écoulement 15 minutes) de manière à ce que la température au sein du réacteur ne soit pas supérieure à -15°C. Comme dans l'exemple 1, le milieu réactionnel est hétérogène dans les premiers instants (précipitation partielle de NH$_4$Cl) et conduit à une concentration en NH$_2$Cl de 2,19 mol L$^{-1}$ (11,3 %) soit un rendement de 94 %.

## EXEMPLE IV :

Pour effectuer la préparation en milieu monophasique avec une eau de Javel à +15°C tout en neutralisant la totalité des ions HO$^-$, un mélange quaternaire NH$_4$Cl-NH$_4$NO$_3$-NH$_3$-H$_2$O a été employé. Sa composition en NH$_3$, NH$_4$Cl, NH$_4$NO$_3$ et H$_2$O est respectivement de 13,14 mol L$^{-1}$ (22,4 %), 3,02 mol L$^{-1}$ (16,2 %), 1,76 mol L$^{-1}$ (14,1 %) et 26,2 mol L$^{-1}$ (47,3 %). Les conditions expérimentales sont identiques à l'exemple 3. Avec une eau de Javel à 98,4 degrés chlorométriques (4,39 mol L$^{-1}$), on obtient une solution de NH$_2$Cl titrant 2,01 mol L$^{-1}$ (rendement 90 %) soit une composition massique de 10,3 %.

## EXEMPLE V :

Cet essai est identique à l'exemple 4 en remplaçant la totalité du chlorure d'ammonium par le nitrate d'ammonium. Ainsi, en partant d'une eau de Javel à 4,65 mol L$^{-1}$ (104.3 degrés chlorométriques) et d'un mélange ammoniacal 7,55 mol L$^{-1}$ en NH$_3$ (12,9%) et 5 mol L$^{-1}$ en NH$_4$Cl (27 %), le rendement de la réaction s'établit à 95 % ce qui correspond à une solution de NH$_2$Cl de 2,21 mol L$^{-1}$ (11,4 %).

## EXEMPLE VI :

### Synthèse de chloramine H.T. en continu

La synthèse de la chloramine est effectuée dans un réacteur agité continu cylindrique maintenu à une température comprise entre -20 et -30°C. Deux ajutages latéraux en position diamétralement opposée permettent l'introduction en continu des solutions d'hypochlorite H.T. et ammoniacale. Les co-réactifs sont préalablement refroidis par circulation dans deux serpentins hélicoïdaux intégrés dans deux jaquettes thermostatiques cylindriques. Leur composition correspond à celle définie dans l'exemple 4. Les débits massiques sont préréglés de manière que l'injection soit équivolumétrique (4 mL. mn$^{-1}$). Les températures d'injection des réactifs ont été fixées respectivement à 15 et -30°C pour NaOCl et le mélange quaternaire NH$_4$Cl-NH$_4$NO$_3$-NH$_3$-H$_2$O. La température en régime au sein du mélange réactionnel est de -11°C. A la sortie du réacteur, on obtient en continu une solution de NH$_2$Cl titrant 2,07 mol L$^{-1}$ (10,6 %).

### Synthèse du N-amino-3-azabicyclo[3.3.0]octane à partir de chloramine H.T.

Les solutions d'hydrazine (N-amino-3-azabicyclo[3.3.0]octane) ont été préparées par action de 30 mL de NH$_2$Cl refroidie à -15°C sur 130 g d'une solution alcaline hétéroazéotropique (30 % de 3-azabicyclo [3.3.0]octane) contenant 3,8 g d'hydroxyde de sodium. Les solutions de chloramine qui titrent 2,18 et 2,21 mol L$^{-1}$ ont été synthétisées respectivement selon les méthodes décrites dans les exemples 4 et 5. La durée d'écoulement est de 20 minutes et la température du mélange réactionnel est maintenue à 60°C. Dans ces conditions, on obtient des solutions de N-amino-3-azabicyclo[3.3.0]octane qui titrent 0,36 et 0,365 mol L$^{-1}$ (4,34 et 4,42 %), soit un rendement par rapport à NH$_2$Cl compris entre 92 et 93 %.

### Synthèse de la N-amino-2-méthylindoline à partir de chloramine H.T.

De la même manière, en procédant dans les conditions du brevet EP 462 016, on obtient une solution de N-amino-2-méthylindoline par action de chloramine sur une solution alcoolique de 2-méthylindoline.

## Revendications

1. Procédé de synthèse de solution de chloramine de concentration supérieure ou égale à 10,3% **caractérisé en ce que** l'on fait réagir une solution d'hypochlorite de sodium de l'ordre de 100 degrés chlorométriques sur un mélange d'ammoniaque et de sels d'ammonium à une température de réaction comprise entre -5 et -20°C.

2. Procédé selon la revendication 1 **caractérisé en ce que** la préparation en continu en milieu homogène doit être effectuée en présence d'une combinaison ammoniacale à base de nitrate d'ammonium (NH$_3$/(NH$^+_4$)$_\alpha$H$^+_\beta$A$^{(\alpha+\beta)-}$/NH$_4$NO$_3$/H$_2$O), de préférence NH$_3$/NH$_4$NO$_3$/H$_2$O ou NH$_3$/NH$_4$Cl/NH$_4$NO$_3$/H$_2$O.

3. Procédé selon la revendication 1 **caractérisé en ce que** la préparation en discontinu est réalisée avec des mélanges de type $NH_3/(NH^+_4)_\alpha H_\beta^+ A^{(\alpha+\beta)-}/H_2O$, de préférence $NH_3/NH_4Cl/H_2O$.

4. Procédé selon les revendications 2 ou 3 **caractérisé en ce que** la température d'addition de l'hypochlorite de sodium à 100 degrés chlorométriques est supérieure ou égale à 15°C.

5. Procédé selon la revendication 1 **caractérisé en ce que** le réactif ammoniacal est refroidi avant réaction à une température comprise entre -20 et -30°C.

6. Procédé selon la revendication 1 **caractérisé en ce que** 90 % au moins des ions hydroxyle produits par la réaction sont neutralisés et que le pH est maintenu entre 10 et 12, de préférence au voisinage de 10,5.

7. Procédé selon la revendication 1 **caractérisé en ce que** le rapport des réactifs $([NH_3]+[NH^+_4])/[ClO^-]$ est compris entre 2 et 5, de préférence autour de 2,7.

**Claims**

1. Process for the synthesis of chloramine solution having a concentration greater than or equal to 10.3 %, **characterised in that** a sodium hypochlorite solution of the order of 100 chlorometric degrees is reacted with a mixture of ammonia and ammonium salts at a reaction temperature of from -5 to -20°C.

2. Process according to claim 1, **characterised in that** continuous preparation in a homogeneous medium must be carried out in the presence of an ammoniacal combination based on ammonium nitrate $(NH_3/(NH^+_4)_\alpha H^+_\beta A^{(\alpha+\beta)-}/NH_4NO_3/H_2O)$, preferably $NH_3/NH_4NO_3/H_2O$ or $NH_3/NH_4Cl/NH_4NO_3/H_2O$.

3. Process according to claim 1, **characterised in that** discontinuous preparation is carried out using mixtures of the type $NH_3/(NH^+_4)_\alpha H_\beta^+ A^{(\alpha+\beta)-}/H_2O$, preferably $NH_3/NH_4Cl/H_2O$.

4. Process according to claim 2 or 3, **characterised in that** the temperature of addition of the sodium hypochlorite of 100 chlorometric degrees is higher than or equal to 15°C.

5. Process according to claim 1, **characterised in that** the ammoniacal reagent is cooled to a temperature of from -20 to -30°C before reaction.

6. Process according to claim 1, **characterised in that** at least 90 % of the hydroxyl ions produced by the reaction are neutralised and the pH is maintained at from 10 to 12, preferably at approximately 10.5.

7. Process according to claim 1, **characterised in that** the ratio of the reagents $([NH_3]+[NH^+_4])/[ClO^-]$ is from 2 to 5, preferably about 2.7.

**Patentansprüche**

1. Verfahren zur Synthese einer Chloramln-Lösung mit einer Konzentration von größer oder gleich als 10,3 %, **dadurch gekennzeichnet, daß** man eine Natriumhypochlorit-Lösung von etwa 100 chlorometrischen Graden mit einer Mischung aus Ammoniak und Ammoniumsalzen bei einer Reaktionstemperatur zwischen -5 und -20°C umsetzt.

2. Verfahren nach Anspruch 1, **dadurch gekennzeichnet, daß** die kontinuierliche Herstellung in homogenem Medium in Gegenwart einer ammoniakalischen Kombination auf der Grundlage von Ammoniumnitrat $(NH_3/(NH^+_4)_\alpha H^+_\beta A^{(\alpha+\beta)-}/NH_4NO_3/H_2O)$, vorzugsweise $NH_3/NH_4NO_3/H_2O$ oder $NH_3/NH_4Cl/NH_4NO_3/H_2O$ durchgeführt wird.

3. Verfahren nach Anspruch 1, **dadurch gekennzeichnet, daß** die diskontinuierliche Herstellung mit Mischungen des Typs $NH_3/(NH^+_4)_\alpha H_\beta^+ A^{(\alpha+\beta)-}/H_2O$, vorzugsweise $NH_3/NH_4Cl/H_2O$ durchgeführt wird.

4. Verfahren nach den Ansprüchen 2 oder 3, **dadurch gekennzeichnet, daß** die Temperatur der Zugabe von Natriumhypochlorit mit 100 chlorometrischen Graden bei größer oder gleich 15°C erfolgt.

5. Verfahren nach Anspruch 1, **dadurch gekennzeichnet, daß** das Ammoniak-Reagens vor der Reaktion auf eine Temperatur zwischen -20 und -30°C abgekühlt wird.

6. Verfahren nach Anspruch 1, **dadurch gekennzeichnet, daß** mindestens 90 % der bei der Reaktion gebildeten Hydroxylionen neutralisiert werden und der pH-Wert zwischen 10 und 12, vorzugsweise bei etwa 10,5 gehalten wird.

7. Verfahren nach Anspruch 1, **dadurch gekennzeichnet, daß** das Verhältnis der Reaktionsteilnehmer $([NH_3]+[NH^+_4])/[ClO^-]$ zwischen 2 und 5 und vorzugsweise bei etwa 2,7 liegt.